# EUROPÄISCHE PATENTANMELDUNG

(11) **EP 0 545 204 A1**
(43) Veröffentlichungstag der Anmeldung: **09.06.1993**
(21) Anmeldenummer: 92119921.2
(22) Anmeldetag: 23.11.1992
(51) Int. Cl.: C07D 491/056, A01N 43/90

(54) **2-Cyanobenzimidazole, ein Verfahren zu ihrer Herstellung und ihre Verwendung in der Landwirtschaft und neue Vorprodukte**

(30) Priorität: 04.12.1991 DE 4139950
(71) Anmelder: BAYER AG, 51368 Leverkusen (DE)
(72) Erfinder: Lunkenheimer, Winfried, Dr., W-5600 Wuppertal (DE); Dehne, Heinz-Wilhelm, Dr., W-4019 Monheim (DE); Wachendorff-BNeumann, Ulrike, Dr., W-4019 Monheim (DE)

(57) **Zusammenfassung**

Neue 2-Cyanobenzimidazole der Formel (I) 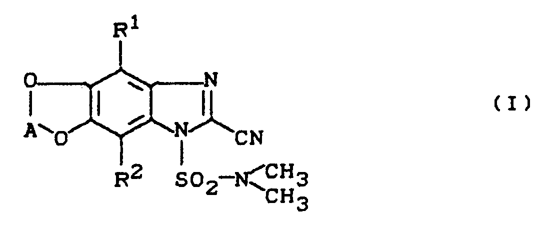
in welcher
R¹, R² und A die in der Beschreibung angegebene Bedeutung haben, ein Verfahren zur Herstellung, ihre Verwendung in der Landwirtschaft z.B. gegen Pilze, Insekten u.a. und neue Zwischenprodukte.

Die Verbindungen sind durch die Formel (I) definiert, man erhält sie z.B. aus geeigneten in 1-Stellung unsubstituierten 2-Cyanobenzimidazolen mit N,N-Dimethylsulfamoylhalogenid. Die in 1-Stellung unsubstituierten 2-Cyanobenzimidazole sind ebenfalls neu und können aus geeigneten Phenylendiaminen der Formel (IV) mit 2,2,2-Trichloracetimidsäuremethylester der Formel (V) zu den ebenfalls neuen 2-Trichlormethylbenzimidazolen der Formel (VI) umgesetzt werden, aus denen dann mit Ammoniak die Verbindungen der Formel (II) gewonnen werden.

## Beschreibung

Die Erfindung betrifft neue 2-Cyanbenzimidazole, ein Verfahren zu ihrer Herstellung und ihre Verwendung als Schädlingsbekämpfungsmittel, außerdem neue Ausgangsverbindungen.

Es ist bekannt, daß bestimmte 2-Cyanobenzimidazole wie beispielsweise die Verbindung 5-Chlor-2-cyano-1-(N,N-dimethylsulfamoyl)-benzimidazol oder die Verbindung 6-Chlor-2-cyano-1-(N,N-dimethylsulfamoyl)-benzimidazol oder die Verbindung 5,6-Dichlor-2-cyano-1-(N,N-dimethylsulfamoyl)-benzimidazol fungizide Eigenschaften besitzen (vergleiche z. B. GB 21 14 567).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue 2-Cyanobenzimidazole der allgemeinen Formel (I), 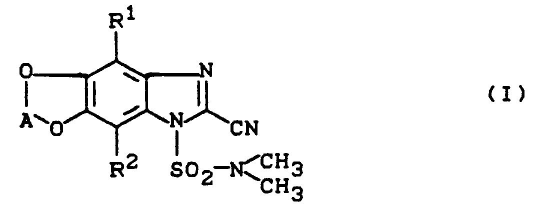
in welcher
- R¹: für Wasserstoff, Halogen oder Alkyl steht,
- R²: für Wasserstoff, Halogen oder Alkyl steht und
- A: für einen gegebenenfalls substituierten, zweifach verknüpften Alkandiylrest steht,
gefunden.

Die Verbindungen der Formel (I) können gegebenenfalls in Abhängigkeit von der Art der Substituenten als optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen 2-Cyanobenzimidazole der allgemeinen Formel (I), 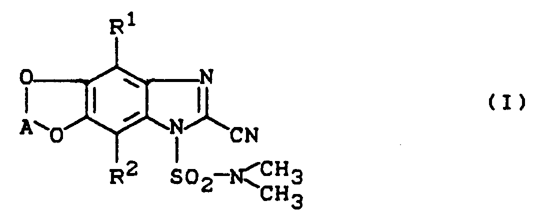
in welcher
- R¹: für Wasserstoff, Halogen oder Alkyl steht,
- R²: für Wasserstoff, Halogen oder Alkyl steht und
- A: für einen gegebenenfalls substituierten, zweifach verknüpften Alkandiylrest steht,
erhält, wenn man in 1-Stellung unsubstituierte 2-Cyanobenzimidazole der Formel (II), 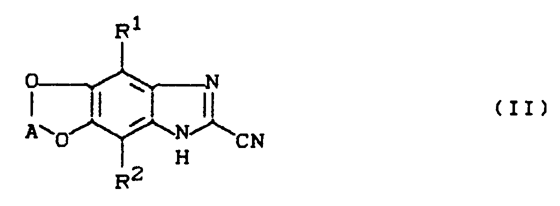
in welcher
R¹, R² und A die oben angegebene Bedeutung haben,
mit N,N-Dimethylsulfamoylchlorid der Formel (III), 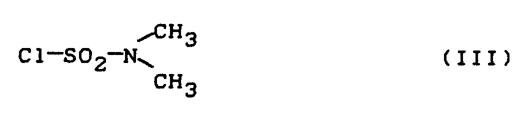
gegebenenfalls in Gegenwart eines Verdünnungsmittels und gegebenenfalls in Gegenwart eines Reaktionshilfsmittels umsetzt.

Schließlich wurde gefunden, daß die neuen 2-Cyanobenzimidazole der allgemeinen Formel (I) eine gute Wirksamkeit gegen Schädlinge besitzen.

Überraschenderweise zeigen die erfindungsgemäßen 2-Cyanobenzimidazole der allgemeinen Formel (I) eine erheblich bessere Wirksamkeit gegenüber phytopathogenen Krankheitserregern und gegenüber pflanzenschädigenden Insekten und Milben als die aus dem Stand der Technik bekannten 2-Cyanobenzimidazole, wie beispielsweise die Verbindung 5-Chlor-2-cyano-1-(N,N-dimethylsulfamoyl)-benzimidazol oder die Verbindung 6-Chlor-2-cyano-1-(N,N-dimethylsulfamoyl)-benzimidazol oder die Verbindung 5,6-Dichlor-2-cyano-1-(N-N-dimethylsulfamoyl)-benzimidazol, welche chemisch und wirkungsmäßig naheliegende Verbindungen sind.

Die erfindungsgemäßen 2-Cyanobenzimidazole sind durch die Formel (I) allgemein definiert. Bevorzugt sind Verbindungen der Formel (I), bei welchen
- R¹: für Wasserstoff, Fluor, Chlor, Brom oder für geradkettiges oder verzweigtes Alkyl mit 1 bis 4 Kohlenstoffatomen steht,
- R²: für Wasserstoff, Fluor, Chlor, Brom oder für geradkettiges oder verzweigtes Alkyl mit 1 bis 4 Kohlenstoffatomen steht und
- A: für einen gegebenenfalls einfach oder mehrfach, gleich oder verschieden substituierten, zweifach verknüpften Alkandiylrest mit 1 bis 4 Kohlenstoffatomen steht, wobei als Substituenten infrage kommen:
Halogen, geradkettiges oder verzweigtes Alkyl mit 1 bis 6 Kohlenstoffatomen, geradkettiges oder verzweigtes Halogenalkyl mit 1 bis 6 Kohlenstoffatomen und 1 bis 13 gleichen oder verschiedenen Halogenatomen oder zweifach verknüpftes Alkandiyl mit 3 bis 7 Kohlenstoffatomen.

Besonders bevorzugt sind Verbindungen der Formel (I), bei welchen
- R¹: für Wasserstoff, Chlor, Brom oder für geradkettiges oder verzweigtes Alkyl mit 1 bis 3 Kohlenstoffatomen steht,
- R²: für Wasserstoff, Chlor, Brom oder für geradkettiges oder verzweigtes Alkyl mit 1 bis 3 Kohlenstoffatomen steht und
- A: für einen gegebenenfalls einfach bis sechsfach, gleich oder verschieden substituierten, zweifach verknüpften Alkandiylrest mit 1 bis 4 Kohlenstoffatomen steht, wobei als Substituenten infrage kommen:
Fluor, Chlor, Brom, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschieden Halogenatomen oder zweifach verknüpftes Alkandiyl mit 3 bis 7 Kohlenstoffatomen.

Ganz besonders bevorzugt sind Verbindungen der Formel (I), bei welchen
- R¹: für Wasserstoff, Chlor, Brom, Methyl oder Ethyl steht,
- R²: für Wasserstoff, Chlor, Brom, Methyl oder Ethyl steht und
- A: für gegebenenfalls einfach bis vierfach, gleich oder verschieden substituiertes Methylen, Ethylen, Propylen oder Butylen steht, wobei als Substituenten jeweils infrage kommen:
Fluor, Chlor, Brom, Methyl, Ethyl, n-oder i-Propyl, Trifluormethyl, Trifluorethyl, Trifluorchlorethyl, Tetrafluorethyl, Pentafluorethyl oder jeweils zweifach verknüpftes 1,4-Butandiyl, 1,5-Pentandiyl oder 1,6-Hexandiyl.

Im einzelnen seien außer den bei den Herstellungsbeispielen genannten Verbindungen die folgenden 2-Cyanobenzimidazole der allgemeinen Formel (I) genannt:

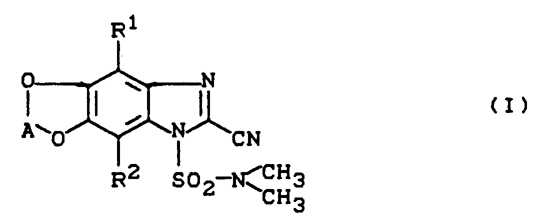

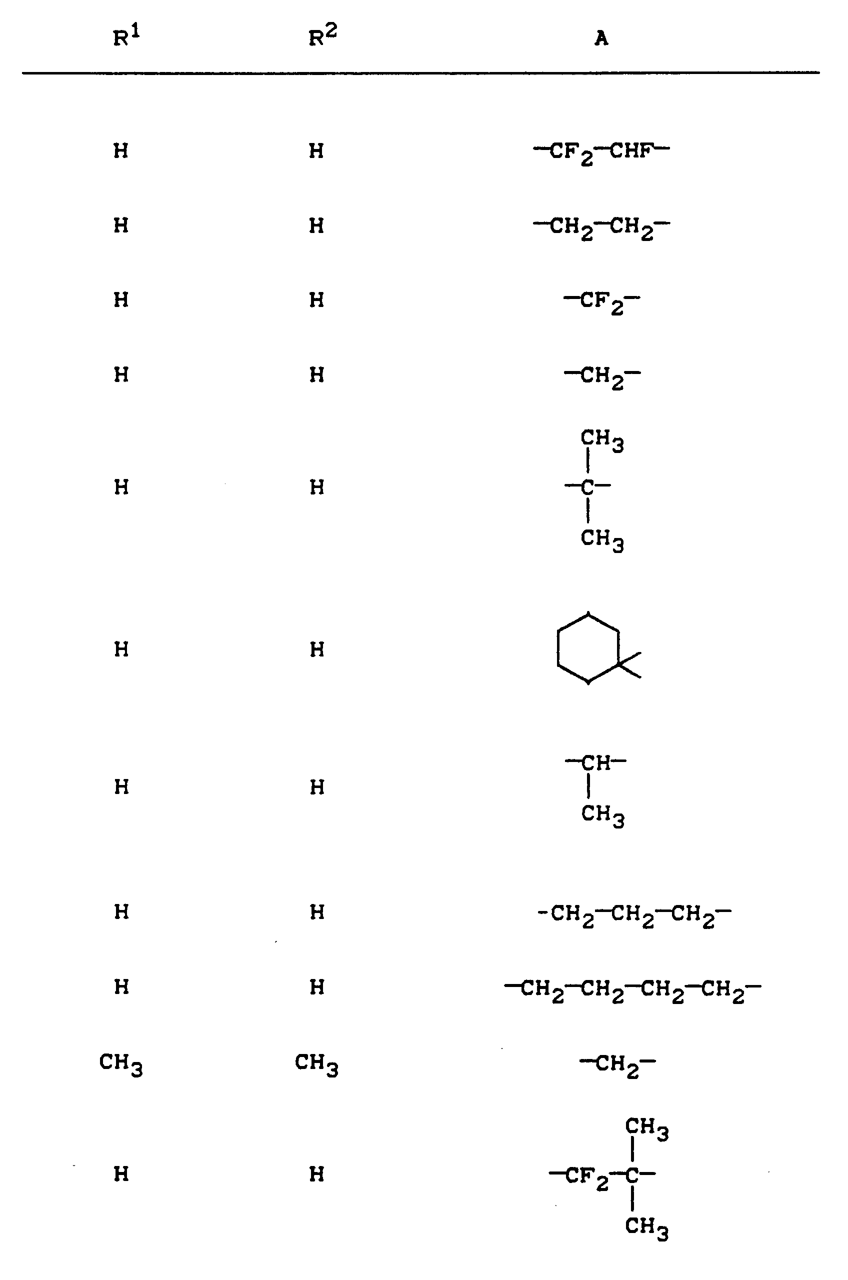

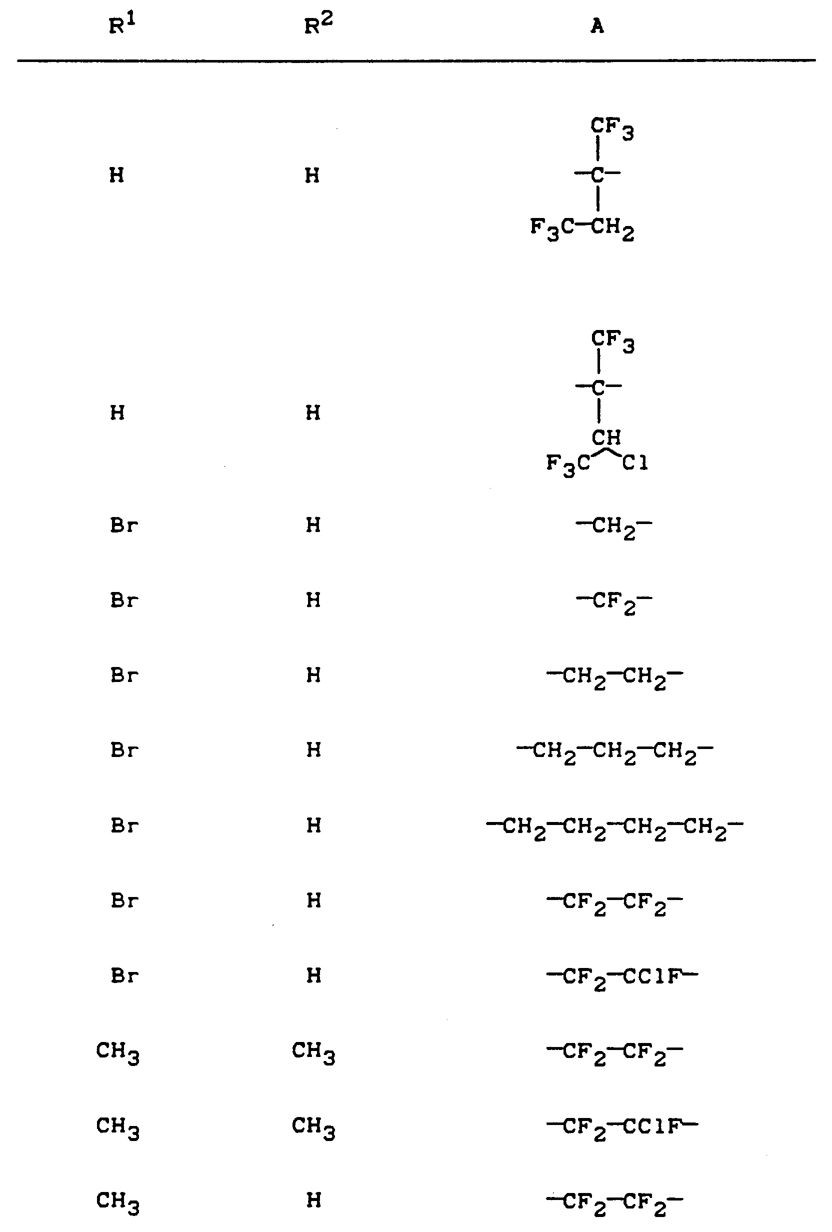

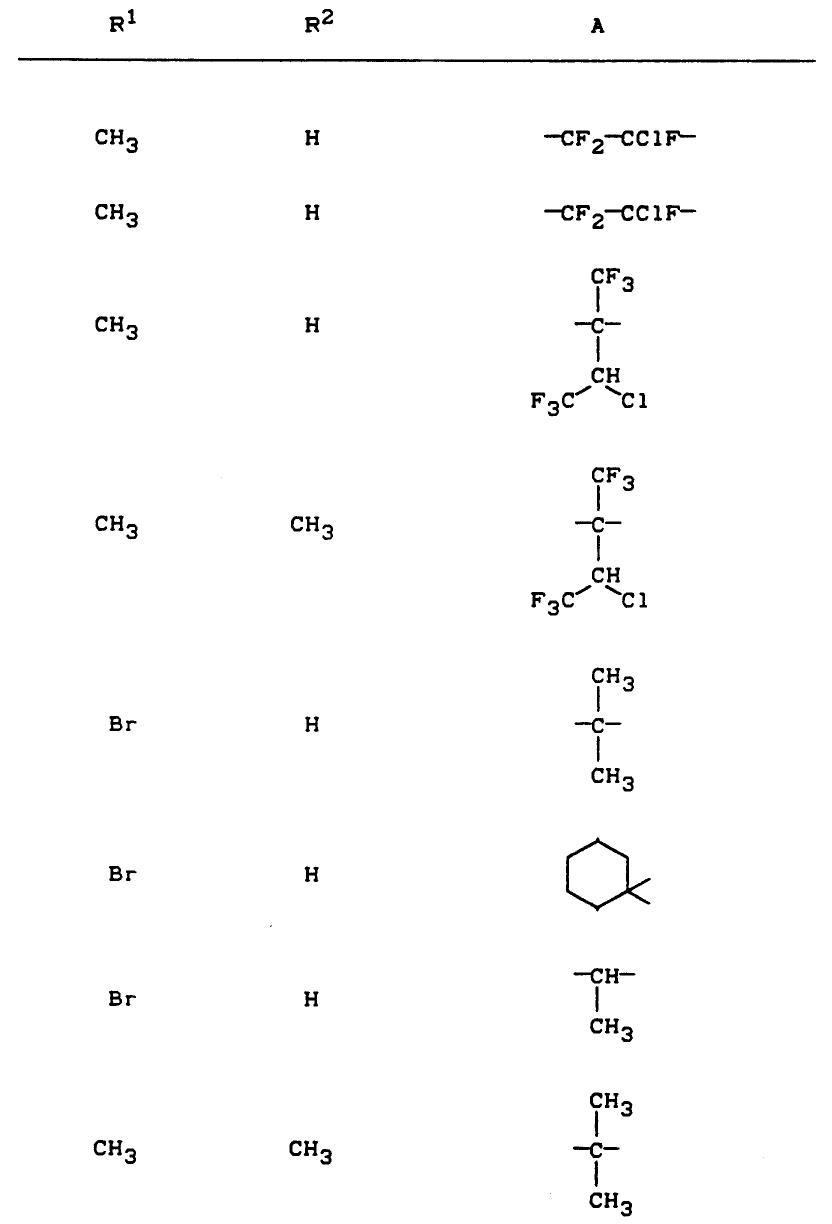

Verwendet man beispielsweise 7-Cyano-2,2,3,3-tetrafluorimidazo[4,5-g]1,4-benzodioxan und N,N-Dimethylsulfamoylchlorid als Ausgangsverbindungen, so läßt sich der Reaktionsablauf des erfindungsgemäßen Verfahrens durch das folgende Formelschema darstellen: 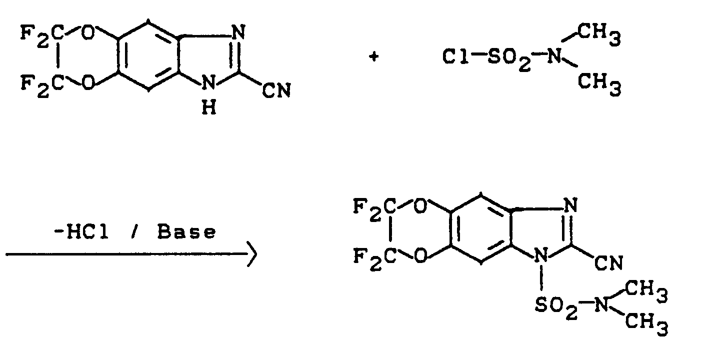
Die zur Durchführung des erfindungsgemäßen Verfahrens als Ausgangsstoffe benötigten in 1-Stellung unsubstituierten 2-Cyanobenzimidazole sind durch die Formel (II) allgemein definiert. In dieser Formel (II) stehen R¹, R² und A vorzugsweise für diejenigen Reste, die bereits im Zusammenhang mit der Beschreibung der erfindungsgemäßen Verbindungen der Formel (I) als bevorzugt für diese Substituenten genannt wurden.
Die in 1-Stellung unsubstituierten 2-Cyanobenzimidazole der Formel (II) sind noch nicht bekannt und ebenfalls Gegenstand der Erfindung. Man erhält sie, wenn man Phenylendiamine der Formel (IV), 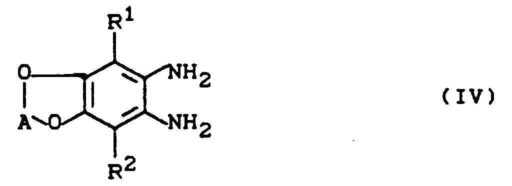
in welcher
R¹, R² und A die oben angegebene Bedeutung haben,
oder deren Säureadditionssalze zunächst in einer ersten Stufe mit 2,2,2-Trichloracetimidsäuremethylester der Formel (V) 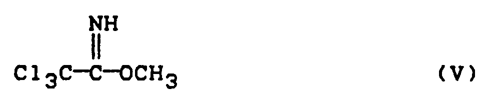
gegebenenfalls in Gegenwart eines Verdünnungsmittels wie beispielsweise Eisessig oder Methanol bei Temperaturen zwischen 0°C und 60°C umsetzt (vergleiche hierzu beispielsweise J. Chem. Soc. C; 1967, 20 oder US 3.576.818 [1967]) und dann die so erhältlichen 2-Trichlormethyl-benzimidazole der Formel (VI), 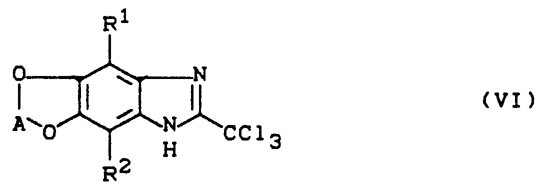
in welcher
R¹, R² und A die oben angegebene Bedeutung haben,
in einer anschließenden zweiten Stufe mit Ammoniak gegebenenfalls in Gegenwart eines Verdünnungsmittels wie beispielsweise Methanol oder Wasser bei Temperaturen zwischen 0°C und 60°C umsetzt (vergleiche hierzu beispielsweise J. Chem. Soc. C; 1967, 25 oder US 3.576.818 [1967]).

2-Trichlormethyl-benzimidazole der Formel (VI) sind noch nicht bekannt und ebenfalls Gegenstand der vorliegenden Erfindung.

Phenylendiamine der Formel (IV) sind bekannt oder erhältlich in Analogie zu allgemein bekannten Verfahren (vergleiche hierzu beispielsweise DE 36 21 301; DE 36 05 977 oder DE 31 32 613).

Als Verdünnungsmittel zur Durchführung des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether wie Diethylether, Diisopropylether, Dioxan, Tetrahydrofuran oder Ethylenglykoldimethyl- oder -diethylether; Ketone wie Aceton oder Butanon oder Methyl-isobutyl-keton; Nitrile wie Acetonitril, Propionitril oder Benzonitril oder Ester wie Essigsäuremethylester oder Essigsäureethylester.

Das erfindungsgemäße Verfahren wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydroxide wie Natriumhydroxid, Calciumhydroxid, Kaliumhydroxid oder auch Ammoniumhydroxid, Alkalimetallcarbonate wie Natriumcarbonat, Kaliumcarbonat, Kaliumhydrogencarbonat, Natriumhydrogencarbonat, Alkali- oder Erdalkalimetallacetate wie Natriumacetat, Kaliumacetat, Calciumacetat sowie tertiäre Amine wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen 0°C und 150°C, vorzugsweise bei Temperaturen zwischen 20°C und 120°C.

Das erfindungsgemäße Verfahren wird üblicherweise unter Normaldruck durchgeführt. Es ist jedoch auch möglich, unter erhöhtem oder vermindertem Druck zu arbeiten.

Zur Durchführung des erfindungsgemäßen Verfahrens setzt man pro Mol an in 1-Stellung unsubstituiertem 2-Cyanobenzimidazol der Formel (II) im allgemeinen 1.0 bis 2.0 Mol, vorzugsweise 1.0 bis 1.3 Mol an N,N-Dimethylsulfamoylchlorid der Formel (III) und gegebenenfalls 1.0 bis 2.0 Mol, vorzugsweise 1.0 bis 1.3 Mol an Reaktionshilfsmittel ein. Die Reaktionsdurchführung, Aufarbeitung und Isolierung der Reaktionsprodukte erfolgt nach bekannten Verfahren (vergleiche auch die Herstellungsbeispiele).

Die Reinigung der Endprodukte der Formel (I) erfolgt mit Hilfe üblicher Verfahren, beispielsweise durch Säulenchromatographie oder durch Umkristallisieren.
Die Charakterisierung erfolgt mit Hilfe des Schmelzpunktes und der Protonen-Kernresonanzspektroskopie (¹H-NMR).

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.
Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei können die erfindungsgemäßen Wirkstoffe mit besonders gutem Erfolg zur Bekämpfung von Krankheiten im Obst- und Gemüseanbau, wie beispielsweise gegen den Erreger der Tomatenbraunfäule (Phytophthora infestans) oder gegen den Erreger des echten Rebenmehltaues (Plasmopara viticola) eingesetzt werden. Sie zeigen bei der Bekämpfung dieser Pflanzenkrankheiten sowohl protektive als auch kurative Eigenschaften.

Darüberhinaus eignen sich die erfindungsgemäßen Wirkstoffe zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam.
Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata,
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp.,
Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp.,
Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
Aus der Ordnung der Arachnida z.B. Scorpio maurus, Lactrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide Wirksamkeit aus.
Sie lassen sich mit besonders gutem Erfolg zur Bekämpfung von pflanzenschädigenden Milben, wie beispielsweise gegen die gemeine Spinnmilbe (Tetranychus urticae) einsetzen. Daneben zeigen die erfindungsgemäßen Wirkstoffe auch blattinsektizide Eigenschaften.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.
Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können bei der Anwendung als Fungizide in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können bei der Anwendung als Fungizide als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen bei der Anwendung als Fungizide in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden bei der Anwendung als Fungizide im allgemeinen Wirkstoffmengen von 0,001 bis 50 g je Kilogramm Saatgut, vorzugsweise 0,01 bis 10 g benötigt.

Bei Behandlung des Bodens sind bei der Anwendung als Fungizide Wirkstoffkonzentrationen von 0,00001 bis 0,1 Gew.-%, vorzugsweise von 0,0001 bis 0,02 % am Wirkungsort erforderlich.

Die erfindungsgemäßen Wirkstoffe können bei der Anwendung als Insektizide und Akarizide in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können bei der Anwendung als Insektizide und Akarizide ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann bei der Anwendung als Insektizide und Akarizide in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die Herstellung und die Verwendung der erfindungsgemäßen Wirkstoffe geht aus den nachfolgenden Beispielen hervor.

### Herstellungsbeispiele

### Beispiel 1

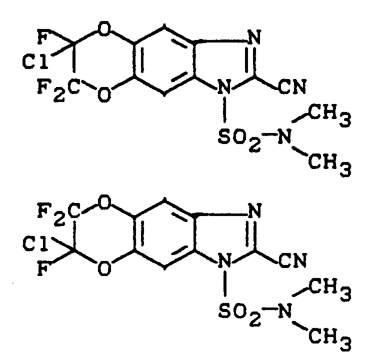

Zu 4,4 g (0,015 Mol) racem. 2-Chlor-7-cyano-2,3,3-trifluor-imidazo[4,5-g]1,4-benzodioxan und 4,2 g (0,03 Mol) pulverisiertem Kaliumcarbonat in 50 ml Acetonitril, gibt man bei Raumtemperatur tropfenweise unter Rühren eine Lösung von 1,6 ml (0,0165 Mol) Dimethylsulfamoylchlorid in 5 ml Acetonitril zu und erhitzt nach beendeter Zugabe für 4 Stunden auf Rückflußtemperatur. Anschließend wird auf Raumtemperatur abgekühlt, filtriert, das Filtrat eingeengt und der Rückstand zwischen Essigester (100 ml) und Wasser (30 ml) verteilt. Die organische Phase wird mit Wasser gewaschen, über Natriumsulfat getrocknet und im Vakuum vom Lösungsmittel befreit.

Man erhält 5,6 g (89 % der Theorie) des oben genannten Gemisches vom Schmelzpunkt 160 °C bis 163 °C.

### Herstellung der Ausgangsverbindung

### Beispiel II-1

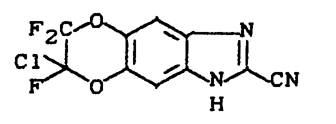

Zu 30 ml konzentriertem wässrigem Ammoniak gibt man bei Raumtemperatur tropfenweise unter Rühren eine Lösung von 3,2 g (0,005 Mol) racem. 2-Chlor-7-trichlormethyl-2,3,3-trifluor-imidazo[4,5-g]1,4-benzodioxan (60-prozentig) in 10 ml Ethanol und rührt nach beendeter Zugabe weitere 4 Stunden bei Raumtemperatur. Zur Aufarbeitung wird mit 20-prozentiger Salzsäure angesäuert, dreimal mit jeweils 30 ml Essigester extrahiert, die vereinigten organischen Phasen getrocknet und im Vakuum eingeengt. Der Rückstand wird über Kieselgel (Laufmittel: Cyclohexan/Essigester 2:1) chromatographiert.

Man erhält 1,1 g (74 % der Theorie) an racem. 2-Chlor-7-cyano-2,3,3-trifluor-imidazo[4,5-g]1,4-benzodioxan vom Schmelzpunkt >230°C.

In entsprechender Weise erhält man außerdem die folgenden Vorprodukte der allgemeinen Formel (II):

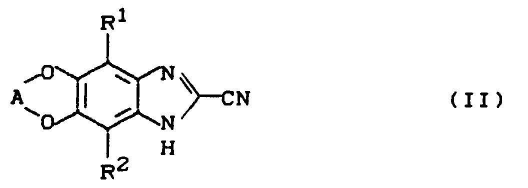

| Bsp. Nr. | A | R¹ | R² | Schmelpunkt °C |
|---|---|---|---|---|
| II-3 | -CH₂-CH₂-CH₂ | H | H | > 230 |
| II-4 | -CF₂- | H | H | > 230 |
| II-5 | -CF₂-CHF- | H | H | ¹H-NMR*): 6,69 (d) |

| | | | | |
|---|---|---|---|---|
| *) Die ¹H-NMR-Spektren wurden in Hexadeutero-Dimethylsulfoxid (DMSO-d₆) mit Tetramethylsilan (TMS) als inneren Standard aufgenommen. Angegeben ist die chemische Verschiebung als δ-Wert in ppm. | | | | |

### Beispiel VI-1

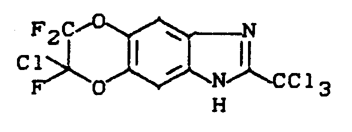

Zu einer Lösung von 7,6 g (0,03 Mol) racem. 2-Chlor-6,7-diamino-2,3,3-trifluor-1,4-benzodioxan (vergleiche z. B. DE 36 05 977) in 60 ml Eisessig gibt man bei Raumtemperatur tropfenweise unter Rühren 8,1 g (0,045 Mol) 2,2,2-Trichloracetimidsäuremethylester, wobei die Temperatur der Reaktionsmischung auf 36°C ansteigt und rührt nach beendeter Zugabe weitere 20 Stunden bei Raumtemperatur. Zur Aufarbeitung gibt man 60 ml Wasser zu, rührt 10 Minuten bei Raumtemperatur, saugt den ausgefallenen Niederschlag ab, wäscht mit Wasser nach und trocknet.

Man erhält 11,4 g (60 % der Theorie) an racem. 2-Chlor-7-trichlormethyl-2,3,3-trifluor-imidazo[4,5-g]1,4-benzodioxan (Gehalt ca. 60 % nach HPLC) vom Schmelzpunkt >230°C.

In entsprechender Weise erhält man außerdem die folgenden Vorprodukte der allgemeinen Formel (VI):

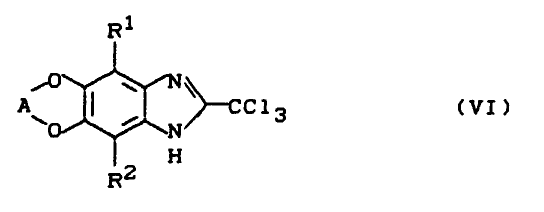

| Bsp. Nr. | A | R¹ | R² | Schmelpunkt °C |
|---|---|---|---|---|
| VI-3 | -CH₂-CH₂-CH₂ | H | H | > 230 |
| VI-4 | -CF₂- | H | H | > 220 |
| VI-5 | -CF₂-CHF- | H | H | 221-225 |

In entsprechender Weise und gemäß den allgemeinen Angaben zur Herstellung erhält man

### Beispiel 2

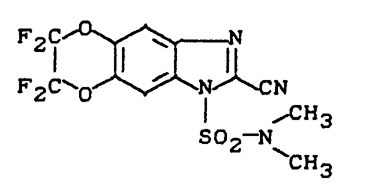

vom Schmelzpunkt 166°C bis 170°C sowie

### Beispiel 3

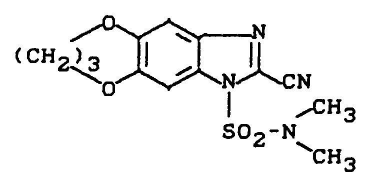

vom Schmelzpunkt 118-120°C.

In entsprechender Weise erhält man die folgenden 2-Cyanobenzimidazole der allgemeinen Formel (I):

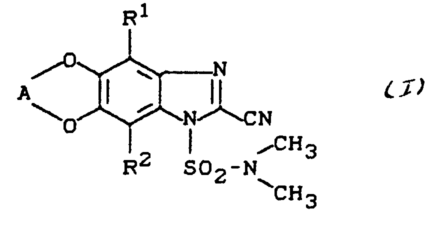

| Bsp. Nr. | A | R¹ | R² | Schmelpunkt °C |
|---|---|---|---|---|
| 4 | -CF₂- | H | H | 206-208 |
| 5 | -CF₂-CHF-(-CHF-CF₂-) | H | H | 110-114 |

### Anwendungsbeispiele:

In den folgenden Anwendungsbeispielen wurden die nachstehend aufgeführten Verbindungen als Vergleichssubstanzen eingesetzt: 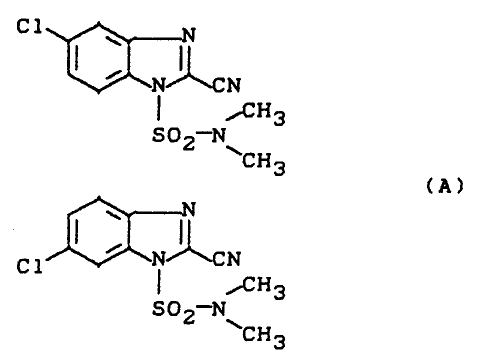
5(6)-Chlor-2-cyano-1-(N,N-dimethylsulfamoyl)-benzimidazol 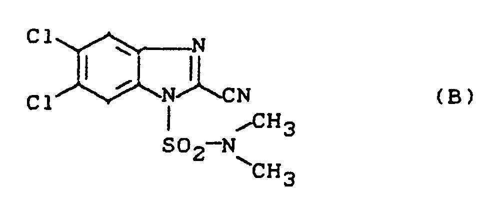
5,6-Dichlor-2-cyano-1-(N,N-dimethylsulfamoyl)-benzimidazol
(beide bekannt aus GB 21 14 567)

### Beispiel A

### Phytophthora-Test (Tomate) / kurativ

- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Sporensuspension von Phytophthora infestans inokuliert. Die Pflanzen verbleiben 7 Stunden bei 20°C und 100 % relativer Luftfeuchtigkeit in einer Inkubationskabine. Nach einer kurzen Antrocknungszeit werden die Pflanzen mit der Wirkstoffzubereitung tropfnaß gespritzt.

Die Pflanzen werden in einer Inkubationskabine mit 100% relativer Luftfeuchtigkeit und ca. 20 °C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen: 1 und 2.

### Beispiel B

### Plasmopara-Test (Reben) / protektiv

- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22 °C und 100 % relativer Luftfeuchtigkeit. Anschließend werden die Pflanzen 5 Tage im Gewächshaus bei 21 °C und ca. 90 % Luftfeuchtigkeit aufgestellt. Die Pflanzen werden dann angefeuchtet und 1 Tag in eine Feuchtkammer gestellt.

6 Tage nach der Inokulation erfolgt die Auswertung.

Eine deutliche Überlegenheit in der Wirksamkeit gegenüber dem Stand der Technik zeigen in diesem Test z. B. die Verbindungen gemäß den Herstellungsbeispielen: 1 und 2

### Beispiel C

### Plasmopara-Test (Reben) / kurativ

- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wässrigen Sporensuspension von Plasmopara viticola inokuliert. Die Pflanzen verbleiben 24 Stunden in einer Feuchtkammer bei 20°C bis 22°C und 100 % relativer Luftfeuchtigkeit und nach weiteren 24 Stunden bei 21°C und 90 % Luftfeuchtigkeit bespritzt man die Pflanzen mit der Wirkstoffzubereitung bis zur Tropfnässe. Anschließend werden die Pflanzen 5 Tage im Gewächshaus aufgestellt. Die Pflanzen werden dann angefeuchtet und einen Tag in eine Feuchtkammer gestellt.

6 Tage nach der Inokulation erfolgt die Auswertung.

Eine deutliche Überlegenheit in der Wirksamkeit gegenüber dem Stand der Technik zeigen in diesem Test z. B. die Verbindungen gemäß den Herstellungsbeispielen: 1 und 2
* organische Phosphorsäureester

### Beispiel D

### Tetranychus-Test (OP*-resistent)

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Bohnenpflanzen (Phaseolus vulgaris), die stark von allen Entwicklungsstadien der gemeinen Spinnmilbe (Tetranychus urticae) befallen sind, werden in eine Wirkstoffzubereitung der gewünschten Konzentration getaucht.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z. B. die Verbindungen der Herstellungsbeispiele 1 und 2 überlegene Wirksamkeit gegenüber dem Stand der Technik,

## Patentansprüche

1. 2-Cyanobenzimidazole der allgemeinen Formel (I), 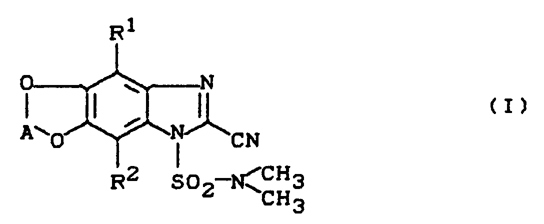 in welcher
R¹ für Wasserstoff, Halogen oder Alkyl steht,
R² für Wasserstoff, Halogen oder Alkyl steht und
A für einen gegebenenfalls substituierten, zweifach verknüpften Alkandiylrest steht.

2. 2-Cyanobenzimidazole der allgemeinen Formel (I) gemäß Anspruch 1,
in welcher
R¹ für Wasserstoff, Fluor, Chlor, Brom oder für geradkettiges oder verzweigtes Alkyl mit 1 bis 4 Kohlenstoffatomen steht,
R² für Wasserstoff, Fluor, Chlor, Brom oder für geradkettiges oder verzweigtes Alkyl mit 1 bis 4 Kohlenstoffatomen steht und
A für einen gegebenenfalls einfach oder mehrfach, gleich oder verschieden substituierten, zweifach verknüpften Alkandiylrest mit 1 bis 4 Kohlenstoffatomen steht, wobei als Substituenten infrage kommen:
Halogen, geradkettiges oder verzweigtes Alkyl mit 1 bis 6 Kohlenstoffatomen, geradkettiges oder verzweigtes Halogenalkyl mit 1 bis 6 Kohlenstoffatomen und 1 bis 13 gleichen oder verschiedenen Halogenatomen oder zweifach verknüpftes Alkandiyl mit 3 bis 7 Kohlenstoffatomen.

3. 2-Cyanobenzimidazole der allgemeinen Formel (I) gemäß Anspruch 1,
in welchen
R¹ für Wasserstoff, Chlor, Brom oder für geradkettiges oder verzweigtes Alkyl mit 1 bis 3 Kohlenstoffatomen steht,
R² für Wasserstoff, Chlor, Brom oder für geradkettiges oder verzweigtes Alkyl mit 1 bis 3 Kohlenstoffatomen steht und
A für einen gegebenenfalls einfach bis sechsfach, gleich oder verschieden substituierten, zweifach verknüpften Alkandiylrest mit 1 bis 4 Kohlenstoffatomen steht, wobei als Substituenten infrage kommen:
Fluor, Chlor, Brom, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschieden Halogenatomen oder zweifach verknüpftes Alkandiyl mit 3 bis 7 Kohlenstoffatomen.

4. 2-Cyanobenzimidazole der allgemeinen Formel (I) gemäß Anspruch 1,
in welchen
R¹ für Wasserstoff, Chlor, Brom, Methyl oder Ethyl steht,
R² für Wasserstoff, Chlor, Brom, Methyl oder Ethyl steht und
A für gegebenenfalls einfach bis vierfach, gleich oder verschieden substituiertes Methylen, Ethylen, Propylen oder Butylen steht, wobei als Substituenten jeweils infrage kommen:
Fluor, Chlor, Brom, Methyl, Ethyl, n-oder i-Propyl, Trifluormethyl, Trifluorethyl, Trifluorchlorethyl, Tetrafluorethyl, Pentafluorethyl oder jeweils zweifach verknüpftes 1,4-Butandiyl, 1,5-Pentandiyl oder 1,6-Hexandiyl.

5. Verfahren zur Herstellung von 2-Cyanobenzimidazolen der allgemeinen Formel (I) 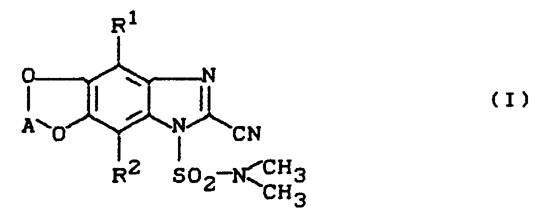 in welcher
R¹ für Wasserstoff, Halogen oder Alkyl steht,
R² für Wasserstoff, Halogen oder Alkyl steht und
A für einen gegebenenfalls substituierten, zweifach verknüpften Alkandiylrest steht,
dadurch gekennzeichnet, daß man in 1-Stellung unsubstituierte 2-Cyanobenzimidazole der Formel (II), 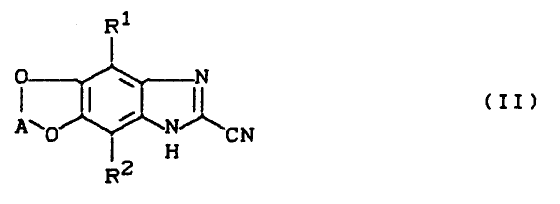 in welcher
R¹, R² und A die oben angegebene Bedeutung haben,
mit N,N-Dimethylsulfamoylchlorid der Formel (III), 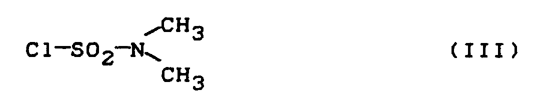 gegebenenfalls in Gegenwart eines Verdünnungsmittels und gegebenenfalls in Gegenwart eines Reaktionshilfsmittels umsetzt.

6. Schädlingsbekämpfungsmittel, gekennzeichnet durch einen Gehalt an mindestens einem 2-Cyanobenzimidazol der Formel (I) nach den Ansprüchen 1 und 5.

7. Verwendung von 2-Cyanobenzimidazol der Formel (I) nach den Ansprüchen 1 und 5 zur Bekämpfung von Schädlingen.

8. Verfahren zur Bekämpfung von Schädlingen, dadurch gekennzeichnet, daß man mindestens ein 2-Cyanobenzimidazol der Formel (I) nach den Ansprüchen 1 und 5 auf Schädlinge und/oder ihren Lebensraum einwirken läßt.

9. Verfahren zur Herstellung von Schädlingsbekämpfungsmitteln, dadurch gekennzeichnet, daß man 2-Cyanobenzimidazole der Formel (I) nach den Ansprüchen 1 und 5 mit Streckmitteln und/oder oberflächenaktiven Mitteln vermischt.

10. In 1-Stellung unsubstituierte 2-Cyanobenzimidazole der Formel (II) 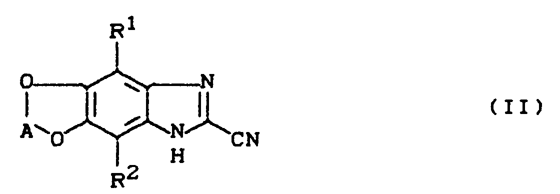 in welcher
R¹ für Wasserstoff, Halogen oder Alkyl steht,
R² für Wasserstoff, Halogen oder Alkyl steht und
A für einen gegebenenfalls substituierten, zweifach verknüpften Alkandiylrest steht.

11. Verfahren zur Herstellung von in 1-Stellung unsubstituierten 2-Cyanobenzimidazolen der Formel (II) 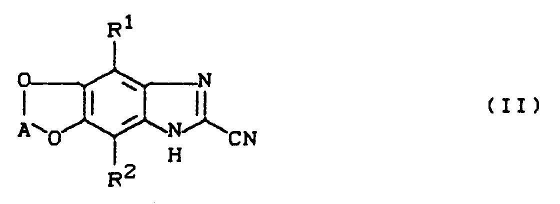 in welcher
R¹ für Wasserstoff, Halogen oder Alkyl steht,
R² für Wasserstoff, Halogen oder Alkyl steht und
A für einen gegebenenfalls substituierten, zweifach verknüpften Alkandiylrest steht,
dadurch gekennzeichnet, daß man Phenylendiamine der Formel (IV) 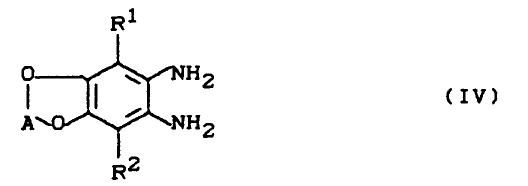 in welcher
R¹, R² und A die oben angegebene Bedeutung haben, oder deren Säureadditionssalze zunächst in einer ersten Stufe mit 2,2,2-Trichloracetimidsäuremethylester der Formel (V) 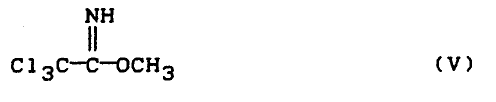 gegebenenfalls in Gegenwart eines Verdünnungsmittels, bei Temperaturen zwischen 0° C und 60° C umsetzt und dann die so erhältlichen 2-Trichlormethyl-benzimidazole der Formel (VI), 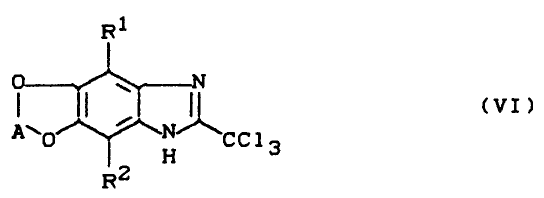 in welcher
R¹, R² und A die oben angegebene Bedeutung haben,
in einer anschließenden zweiten Stufe mit Ammoniak gegebenenfalls in Gegenwart eines Verdünnungsmittels bei Temperaturen zwischen 0⁰ C und 60⁰ C umsetzt.

12. 2-Trichlormethylbenzimidazole der Formel (VI) 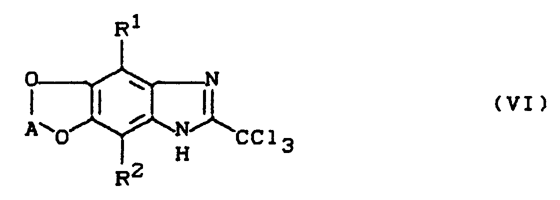 in welcher
R¹ für Wasserstoff, Halogen oder Alkyl steht,
R² für Wasserstoff, Halogen oder Alkyl steht und
A für einen gegebenenfalls substituierten, zweifach verknüpften Alkandiylrest steht.

13. Verfahren zur Herstellung von 2-Trichlormethylbenzimidazolen der Formel (VI) 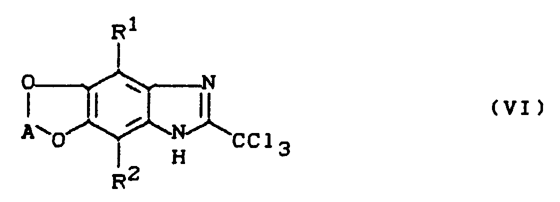 in welcher
R¹ für Wasserstoff, Halogen oder Alkyl steht,
R² für Wasserstoff, Halogen oder Alkyl steht und
A für einen gegebenenfalls substituierten, zweifach verknüpften Alkandiylrest steht,
dadurch gekennzeichnet, daß man Phenylendiamine der Formel (IV) 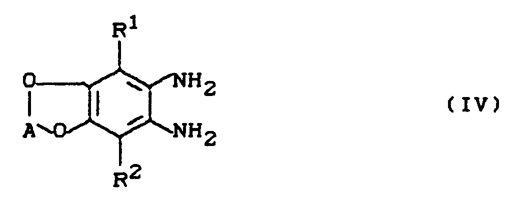 in welcher
R¹, R² und A die oben angegebene Bedeutung haben, oder deren Säureadditionssalze mit 2,2,2-Trichloracetimidsäuremethylester der Formel (V) 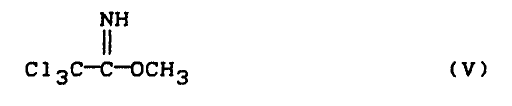 gegebenenfalls in Gegenwart eines Verdünnungsmittels, bei Temperaturen zwischen 0° C und 60° C umsetzt.
